(19) Europäisches Patentamt
European Patent Office
Office européen des brevets

(11) **EP 4 268 231 B1**

(12) **EUROPEAN PATENT SPECIFICATION**

(45) Date of publication and mention
of the grant of the patent:
**05.11.2025 Bulletin 2025/45**

(21) Application number: **21920475.7**

(22) Date of filing: **30.04.2021**

(51) International Patent Classification (IPC):
*G16B 40/00* (2019.01)    *G16B 20/20* (2019.01)
*C12Q 1/6806* (2018.01)    *C12Q 1/6886* (2018.01)

(52) Cooperative Patent Classification (CPC):
(C-Sets available)
**C12Q 1/6886; C12Q 1/6806; G16B 20/20;**
**G16B 40/00;** C12Q 2600/154      (Cont.)

(86) International application number:
**PCT/CN2021/091761**

(87) International publication number:
**WO 2022/156089 (28.07.2022 Gazette 2022/30)**

(54) **DNA METHYLATION SEQUENCING ANALYSIS METHODS**

VERFAHREN ZUR ANALYSE DER DNA-METHYLIERUNGSSEQUENZIERUNG

MÉTHODES D'ANALYSE PAR SÉQUENÇAGE DE LA MÉTHYLATION DE L'ADN

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **20.01.2021 CN 202110072090**
            **21.01.2021 CN 202110078570**

(43) Date of publication of application:
**01.11.2023 Bulletin 2023/44**

(73) Proprietors:
• **Genecast (Taizhou) Biotechnology Co., Ltd.**
**Taizhou (CN)**
• **Genecast (Wuhan) Precision Diagnostic Co., Ltd.**
**Wuhan, Hubei (CN)**

(72) Inventors:
• **HAN, Tiancheng**
**Beijing 100089 (CN)**
• **SONG, Xiaofeng**
**Beijing 100089 (CN)**
• **YU, Jianing**
**Beijing 100089 (CN)**
• **HONG, Yuanyuan**
**Beijing 100089 (CN)**
• **HE, Ji**
**Beijing 100089 (CN)**
• **CHEN, Weizhi**
**Beijing 100089 (CN)**
• **DU, Bo**
**Beijing 100089 (CN)**

(74) Representative: **Frick, Robert**
**Lorenz Seidler Gossel**
**Rechtsanwälte Patentanwälte**
**Partnerschaft mbB**
**Widenmayerstraße 23**
**80538 München (DE)**

(56) References cited:
WO-A1-2018/161031      WO-A1-2020/243609
CN-A- 110 168 099      CN-A- 111 910 004
CN-A- 112 397 150      CN-A- 112 397 151

• **KRISTINA WARTON ET AL: "Methylated**
**circulating tumor DNA in blood: power in cancer**
**prognosis and response", ENDOCRINE**
**RELATED CANCER, vol. 23, no. 3, 13 January**
**2016 (2016-01-13), GB, pages R157 - R171,**
**XP055723832, ISSN: 1351-0088, DOI: 10.1530/**
**ERC-15-0369**

- LIU L. ET AL: "Targeted methylation sequencing of plasma cell-free DNA for cancer detection and classification", vol. 29, no. 6, 1 June 2018 (2018-06-01), pages 1445 - 1453, XP055910054, Retrieved from the Internet <URL:https://www.ncbi.nlm.nih.gov/pmc/articles/PMC6005020/pdf/mdy119.pdf> DOI: 10.1093/annonc/mdy119
- XIAOKE HAO, HUIYAN LUO, MICHAL KRAWCZYK, WEI WEI, WENQIU WANG, JUAN WANG, KEN FLAGG, JIAYI HOU, HENG ZHANG, SHAOHUA YI, MARYAM JAF: "DNA methylation markers for diagnosis and prognosis of common cancers", PROCEEDINGS OF THE NATIONAL ACADEMY OF SCIENCES, vol. 114, no. 28, 11 July 2017 (2017-07-11), pages 7414 - 7419, XP055668394, ISSN: 0027-8424, DOI: 10.1073/pnas.1703577114

(52) Cooperative Patent Classification (CPC): (Cont.)

C-Sets
**C12Q 1/6806, C12Q 2523/125;
C12Q 1/6806, C12Q 2523/125, C12Q 2535/122**

**Description**

BACKGROUND OF THE INVENTION

DNA methylation is an epigenetic mechanism that occurs due to the addition of a methyl group to DNA, thereby modifying the function of genes and affecting gene expression. In some genomic regions, the methylation statuses of neighboring CpG sites are highly correlated. As a result, the methylation status of a single fragment on these sites is usually consistent with the neighboring sites.

In bisulfite or enzymatic conversion of DNA, only unmethylated cytosine (C) is converted into uracil (U). After PCR amplification of converted DNA, the unmethylated Cs of the template fragments become thymines (Ts) in the amplified DNA, and the methylated Cs of the template fragments remain as Cs in the amplified DNA. Thus, the methylation status of CpG sites can be distinguished by conversion and amplification.

Methylation levels can be measured as a ratio called the beta-value, which is the number of Cs divided by the number of Cs plus Ts on this site. Ideally, if the unmethylated Cs are completely converted, the beta-value would be precisely calculated. However, the conversion rate is not 100%, and there also may be sequencing errors during amplification. Typically, then, beta-values are biased, which then leads to biased predictions based on the beta-values.

Cell-free DNA (cfDNA) comprises highly degraded DNA fragments, which are detectable in the peripheral blood of every human. In healthy individuals, the vast majority of cfDNA is derived from the hematopoietic system. In cancer patients, cfDNA includes circulating tumor DNA (ctDNA) shed from tumor cells into the circulation. These fragments retain cancer-specific marks from the originating cancer cells. Therefore, analyses of cfDNA can be used to diagnose cancer at an early stage or monitor minimal residual disease.

However, for ctDNA cancer models, the bias of beta-values significantly interferes with analysis since the ctDNA fraction (CTDF) of plasma/body fluid is low, and beta-values can be greatly distorted by noise. Bias of beta-values also impacts analysis of DNA samples from body fluids and tissues. From WO2018/161031A1 a method for detecting methylation markers is known. Further prior art is the publication by KRISTINA WARTON ET AL, "Methylated circulating tumor DNA in blood: power in cancer prognosis and response", ENDOCRINE RELATED CANCER, vol. 23, no. 3, 13 January 2016 (2016-01-13), pages R157-R171. Further of interest is the publication LIU L. ET AL: "Targeted methylation sequencing of plasma cell-free DNA for cancer detection and classification", vol. 29, no. 6, 1 June 2018 (2018-06-01), pages 1445-1453.

Thus, there is a need for better methods to analyze DNA with less bias.

BRIEF SUMMARY OF THE INVENTION

The invention is defined by the independent claims. Dependent claims recite further embodiments of the invention. In embodiments, the invention provides a method for determining a methylation score of DNA, the method comprising: (a) providing a sample from a subject; (b) isolating DNA from the sample of (a); (c) treating isolated DNA of (b) with bisulfite or enzyme to perform conversion of unmethylated cytosines in the DNA; (d) performing library construction of the converted DNA of (c) by paired end next generation sequencing (NGS); (e) obtaining sequencing data from the paired end NGS of (d) and determining DNA sequences of DNA fragments present, wherein the sequence of a DNA fragment is determined by merging the sequences of read-pairs for the DNA fragment; (f) identifying methylation status of DNA fragments of (e) by comparing a reference genome to the sequencing data of (e) to determine if a cytosine base in a CpG site within a DNA fragment is methylated or unmethylated; (g) calculating for Methylation-Correlated Blocks (MCBs) a Methylated Fragment Ratio (MFR) value or an Unmethylated Fragment Ratio (UFR) value or both a MFR value and an UFR value, wherein the MCBs are based on CpGs pre-determined within the DNA; and (h) calculating a p-value for each of selected differential MCBs, wherein the selected differential MCBs are selected based on pre-determined MFR and UFR values, and wherein the p-value is based on a pre-determined baseline distribution of MFR values if selected differential MCBs are hypermethylated or UFR values if selected differential MCBs are hypomethylated; and (i) calculating a methylation score using the equation

$$Score_n = \frac{-2 \sum_{j=1}^{J} c_j \cdot \ln p_{n,j}}{\sum_{j=1}^{J} c_j}$$

wherein $c_j$ is the weight of $MCB_j$ within $sample_n$, $p_{n,j}$ is the p-value of (h) for $MCB_j$ in $sample_n$, wherein $sample_n$ has $J$ number of MCB, and wherein the methylation score is a hypermethylation score if selected differential MCBs in (h) are hypermethylated and is a hypomethylation score if selected differential MCBs in (h) are hypomethylated and is a hybrid methylation score if selected differential MCBs in (h) comprise both hypermethylated and hypomethylated MCBs.

In embodiments, the invention provides a method for determining a ctDNA Fraction (CTDF) value, the method comprising: (a) providing a sample from a subject; (b) isolating DNA from the sample of (a); (c) treating isolated DNA of (b) with bisulfite or enzyme to perform conversion of unmethylated cytosines in the DNA; (d) performing library construction of the converted DNA of (c) by paired end next generation sequencing (NGS); (e) obtaining sequencing data from the paired end NGS of (d) and determining DNA sequences of DNA fragments present, wherein the sequence of a DNA fragment is determined by merging the sequences of read-pairs for the DNA fragment; (f) identifying methylation status of DNA fragments of (e) by comparing a reference genome to the sequencing data of (e) to determine if a cytosine base in a CpG site within a DNA fragment is methylated or unmethylated; (g) calculating for Methylation-Correlated Blocks (MCBs) a Methylated Fragment Ratio (MFR) value, wherein the MCBs are based on CpGs pre-determined within the DNA; and (h) calculating tumor and non-tumor likelihood values for each of selected differential MCBs, wherein the selected differential MCBs are selected based on pre-determined MFR values, and wherein the tumor and non-tumor likelihood values are based on a pre-determined beta distribution of MFR values calculated in (g); and (i) calculating a ctDNA Fraction (CTDF) value based on the tumor and non-tumor likelihood values determined in (h) using the equation

$$\log P(F|\theta, M) = \sum_c w_c \cdot \log(\theta \cdot P(f^c|m^T_j) + (1 - \theta) \cdot P(f^c|m^N_j))$$

wherein $j$ is the MCB covered by $f^c$; $P(f^c|m^T_j)$ and $P(f^c|m^N_j)$ are

$$P(f|m^T_j) = \prod_h \frac{B(f_h + \alpha^T_j, 1 - f_h + \beta^T_j)}{B(\alpha^T_j, \beta^T_j)}$$

and

$$P(f|m^N_j) = \prod_h \frac{B(f_h + \alpha^N_j, 1 - f_h + \beta^N_j)}{B(\alpha^N_j, \beta^N_j)}$$

respectively, for a given fragment $f^c$; $\alpha^T_j$, $\beta^T_j$, $\alpha^N_j$ and $\beta^N_j$ are parameters of tumor or normal class beta distributions of MFR on MCB $j$, which is estimated from $m^T_j$ and $m^N_j$; $m^T_j$ is the tumor class methylation pattern on *MCBj* and $m^N_j$ is the normal class methylation pattern on MCB$j$; $f_h$ is 0 or 1, representing methylated or unmethylated status of the CpG site $h$ in fragment $f$; $\theta$ is the CTDF estimated by a grid search; and $w_c$ is the weight assigned for $f^c$.

Additional embodiments are as described herein.

BRIEF DESCRIPTION OF THE DRAWINGS

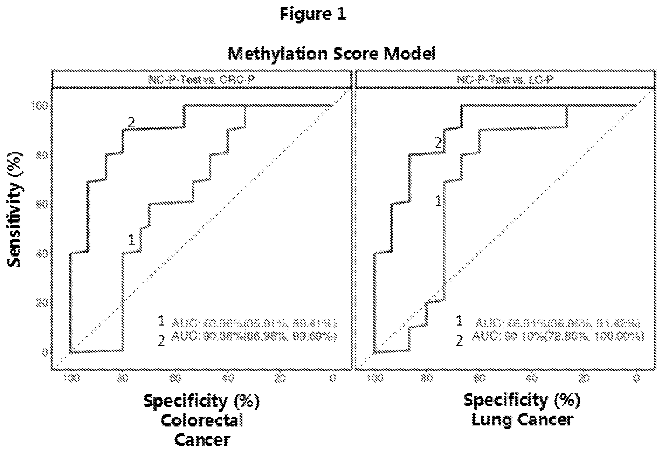

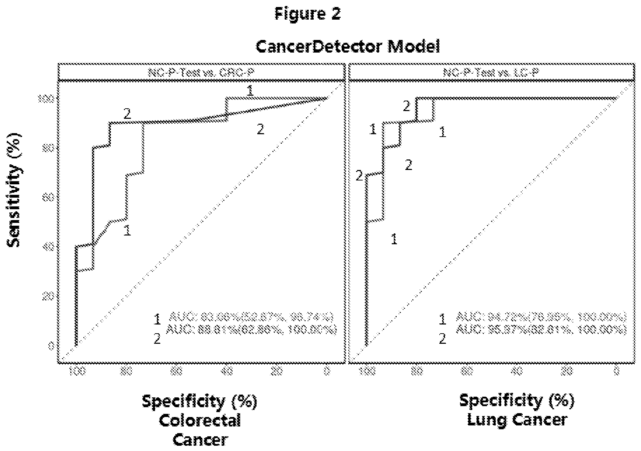

Figures 1 and 2 each present graphs for colorectal cancer (left) and lung cancer (right) plotting sensitivity (%) vs. specificity (%) for comparing models as described in Example 8.
Figure 1 presents comparisons of models of methylation score. Figure 2 presents comparisons of models of CancerDetector.

DETAILED DESCRIPTION OF THE INVENTION

It has been surprisingly and unexpectedly discovered that the methods as described herein improve the performance of epigenetic models. Without wishing to be bound by any theory, the methods as described herein, e.g., suppress noise due to incomplete conversion or sequencing errors by discriminating and removing unreliable reads/fragments and take advantage of the correlation between CpG sites by identifying the true methylation status of a CpG site based on the status of itself and the statuses of its neighboring sites.

In embodiments, the invention provides a method for determining a methylation score of DNA, the method comprising: (a) providing a sample from a subject; (b) isolating DNA from the sample of (a); (c) treating isolated DNA of (b) with bisulfite or enzyme to perform conversion of unmethylated cytosines in the DNA; (d) performing library construction of the converted DNA of (c) by paired end next generation sequencing (NGS); (e) obtaining sequencing data from the paired end NGS of (d) and determining DNA sequences of DNA fragments present, wherein the sequence of a DNA fragment is determined by merging the sequences of read-pairs for the DNA fragment; (f) identifying methylation status of DNA fragments of (e) by comparing a reference genome to the sequencing data of (e) to determine if a cytosine base in a CpG site within a DNA fragment is methylated or unmethylated; (g) calculating for Methylation-Correlated Blocks (MCBs) a Methylated Fragment Ratio (MFR) value or an Unmethylated Fragment Ratio (UFR) value or both a MFR value and an UFR value, wherein the MCBs are based on CpGs pre-determined within the DNA; and (h) calculating a p-value for each of selected differential MCBs, wherein the selected differential MCBs are selected based on pre-determined MFR and UFR values, and wherein the p-value is based on a pre-determined baseline distribution of MFR values if selected differential MCBs are hypermethylated or UFR values if selected differential MCBs are hypomethylated; and (i) calculating a methylation score using the equation

$$Score_n = \frac{-2 \sum_{j=1}^{J} c_j \cdot \ln p_{n,j}}{\sum_{j=1}^{J} c_j}$$

wherein $c_j$ is the weight of $MCB_j$ within $sample_n$, $p_{n,j}$ is the p-value of (h) for $MCB_j$ in $sample_n$, wherein $sample_n$ has $J$ number of MCB, and wherein the methylation score is a hypermethylation score if selected differential MCBs in (h) are hypermethylated and is a hypomethylation score if selected differential MCBs in (h) are hypomethylated and is a hybrid methylation score if selected differential MCBs in (h) comprise both hypermethylated and hypomethylated MCBs.

Bisulfite and enzymatic conversion of DNA for sequencing purposes are well known in the art, and any suitable method may be used. An exemplary method of enzymatic conversion is enzymatic methyl-seq, e.g., commercially available from New England Biolabs as NEBNext® Enzymatic Methyl-Seq (Ipswich, MA, USA).

In embodiments, the selected differential MCBs in (h) are hypermethylated and wherein a hypermethylation score is calculated in (i). In embodiments, the selected differential MCBs in (h) are hypomethylated wherein a hypomethylation score is calculated in (i). In embodiments, the selected differential MCBs in (h) comprise both hypermethylated and hypomethylated MCBs and wherein a hybrid methylation score is calculated in (i).

In embodiments, the $c_j$ is equal to $count_{n,j}$, or $-count_{n,j} \cdot \ln FDR_j$, wherein $count_{n,j}$ is the number of fragments from $sample_n$ on $MCB_j$, and $FDR_j$ is false discovery/positive rate of $MCB_j$. Additional exemplary weights are described within the Examples.

In embodiments, the sample is a plasma sample. In embodiments, the DNA is cell-free DNA.

In principle, the invention provides a method of treating a subject having cancer, the method comprising: (A) determining the methylation score of DNA of a test subject according to a method described above; and (B) determining that the test subject has cancer based on the methylation score of (A); and (C) treating the test subject. This is not subject-matter of the present invention.

Without wishing to be bound by any theory, generally, the methylation score is associated with the fraction of ctDNA fragments in the plasma, when ctDNA is detected. ctDNA fraction in late stage cancer and some specific cancer types tend to be higher, which leads to a higher methylation score.

In embodiments, the invention provides a method for determining a ctDNA Fraction (CTDF) value, the method comprising: (a) providing a sample from a subject; (b) isolating DNA from the sample of (a); (c) treating isolated DNA of (b) with bisulfite or enzyme to perform conversion of unmethylated cytosines in the DNA; (d) performing library construction of the converted DNA of (c) by paired end next generation sequencing (NGS); (e) obtaining sequencing data from the paired end NGS of (d) and determining DNA sequences of DNA fragments present, wherein the sequence of a DNA fragment is determined by merging the sequences of read-pairs for the DNA fragment; (f) identifying methylation status of DNA fragments of (e) by comparing a reference genome to the sequencing data of (e) to determine if a cytosine base in a CpG site within a DNA fragment is methylated or unmethylated; (g) calculating for Methylation-Correlated Blocks (MCBs) a Methylated Fragment Ratio (MFR) value, wherein the MCBs are based on CpGs pre-determined within the DNA; and (h) calculating tumor and non-tumor likelihood values for each of selected differential MCBs, wherein the selected differential MCBs are selected based on pre-determined MFR values, and wherein the tumor and non-tumor likelihood values are based on a pre-determined beta distribution of MFR values calculated in (g); and (i) calculating a ctDNA Fraction (CTDF) value based on the tumor and non-tumor likelihood values determined in (h) using the equation

$$\log P(F|\theta, M) = \sum_c w_c \cdot \log(\theta \cdot P(f^c|m^T{}_j) + (1-\theta) \cdot P(f^c|m^N{}_j))$$

wherein $j$ is the MCB covered by $f^c$; $P(f^c|m^T{}_j)$ and $P(f^c|m^N{}_j)$ are

$$P(f|m^T{}_j) = \prod_h \frac{B(f_h + \alpha^T{}_j, 1 - f_h + \beta^T{}_j)}{B(\alpha^T{}_j, \beta^T{}_j)}$$

and

$$P(f|m^N{}_j) = \prod_h \frac{B(f_h + \alpha^N{}_j, 1 - f_h + \beta^N{}_j)}{B(\alpha^N{}_j, \beta^N{}_j)}$$

respectively, for a given fragment $f^c$; $\alpha^T{}_j$, $\beta^T{}_j$, $\alpha^N{}_j$ and $\beta^N{}_j$ are parameters of tumor or normal class beta distributions of MFR on MCB $j$, which is estimated from $m^T{}_j$ and $m^N{}_j$; $m^T{}_j$ is the tumor class methylation pattern on MCB$j$ and $m^N{}_j$ is the normal class methylation pattern on $MCBj$; $f_h$ is 0 or 1; $\theta$ is estimated by a grid search; and $w_c$ is the weight assigned for $f^c$.

In embodiments, $w_c$ is one of

| |
|---|
| $MR$ |
| $MR^2$ |
| $\sqrt{MR}$ |
| $\dfrac{1}{\log MR}$ |
| $\begin{cases} 1, & MR \geq MR_b \| MR \leq MR_a \\ 0, & MR_a < MR < MR_b \end{cases}$ |

wherein $MR$ is the percentage of methylated CpGs of each fragment, $MR_b$ is the threshold of $MR$ for methylated fragments, and $MR_a$ is the threshold of $MR$ for unmethylated fragments.

In embodiments, the sample is a plasma sample. In embodiments, the DNA is cell-free DNA.

In embodiments, the invention provides a method of treating a subject having cancer, the method comprising: (A) determining the ctDNA Fraction (CTDF) value of a test subject according to a method described above; and (B) determining that the test subject has cancer based on the CTDF of (A); and (C) treating the test subject.

For the inventive methods, pre-determination, as described herein, is based on knowledge of DNA prior to performing the inventive methods and/or is based on knowledge of the results of previously performing the inventive methods on other subjects. The other subjects may be healthy subjects, and the performance of the inventive methods may be to establish baseline values, threshold values, and/or distributions against which to compare future-determined values. For example, MFR and/or UFR values may be established for MCBs of healthy subjects such that certain of the MCBs are deemed differential MCBs based on those values. Also, for example, a distribution of MFR or UFR values may be established for subjects against which another such value may be compared to determine a p-value associated with the value. The Examples provide such exemplary methods.

Methylation scores and CTDF values are each higher in subjects with cancer compared to subjects without cancer. A threshold can be set which depends on the score/value distribution in healthy subjects. Subjects with scores/values higher than the threshold are predicted as diseased. Without prior knowledge of the score/value distribution among cases, the highest score/value in healthy subjects or the 95th percentile can be used as the threshold. The threshold controls the trade-off between sensitivity and specificity (or between positive and negative predictive values (PPV and NPV, respectively)). For example, if the method is applied to cancer diagnoses in high-risk populations, where higher sensitivities are desirable to minimize the number of false negatives, lower thresholds are preferable. Also for example, if the method is applied to screening tests for diseases that are not life-threatening such as prostate cancer, where higher PPVs are desirable to reduce the overtreatment caused by false positives, higher thresholds are preferable. If there is no preference for sensitivity/specificity/PPV/NPV, the optimal threshold can be obtained from a receiver operating characteristic curve (ROC curve). Each point on the curve gives the specificity (1-x) and the sensitivity (y) for a threshold value. The optimal threshold can be represented by either the point closest to the (0, 1) or the one that maximizes the distance from the diagonal line (y=x).

In embodiments, each of the inventive methods can be performed on other DNAs, where the samples used to build the baseline would be changed accordingly. For example, if tissue DNA is tested, baseline samples in the Methylation Score Model and normal baseline samples in the CancerDetector Model would be normal tissues. Thus, blood, biopsy, bodily fluid, and tissues may be samples from which DNA is obtained for the inventive methods.

Sequencing data may be obtained by any suitable next generation sequencing method, e.g., by direct sequencing (e.g., whole genome bisulfite sequencing, WGBS) or hybridization to a pre-designed probe panel to capture the target region for sequencing. Data can also be obtained by Reduced Representation Bisulfite-Sequencing (RRBS), a protocol that uses one or multiple restriction enzymes on the genomic DNA to enrich GC-rich sequence-specific fragmentation. It is more cost-effective than WGBS and covers about 4 million CpGs.

The terms "treat," and "prevent" as well as words stemming therefrom, as used herein, do not necessarily imply 100% or complete treatment or prevention. Rather, there are varying degrees of treatment or prevention of which one of ordinary skill in the art recognizes as having a potential benefit or therapeutic effect. In this respect, the methods can provide any amount or any level of treatment or prevention of cancer in a subject. Furthermore, the treatment or prevention provided by the method can include treatment or prevention of one or more conditions or symptoms of the disease being treated or prevented. Also, for purposes herein, "prevention" can encompass delaying the onset of the disease, or a symptom or condition thereof.

In embodiments, for any of the inventive methods the subject is a human.

The following includes certain aspects of the invention.

1. A method for determining a methylation score of DNA, the method comprising:

(a) providing a sample from a subject;
(b) isolating DNA from the sample of (a);
(c) treating isolated DNA of (b) with bisulfite or enzyme to perform conversion of unmethylated cytosines in the DNA;
(d) performing library construction of the converted DNA of (c) by paired end next generation sequencing (NGS);
(e) obtaining sequencing data from the paired end NGS of (d) and determining DNA sequences of DNA fragments present,
wherein the sequence of a DNA fragment is determined by merging the sequences of read-pairs for the DNA fragment;
(f) identifying methylation status of DNA fragments of (e) by comparing a reference genome to the sequencing data of (e) to determine if a cytosine base in a CpG site within a DNA fragment is methylated or unmethylated;
(g) calculating for Methylation-Correlated Blocks (MCBs) a Methylated Fragment Ratio (MFR) value or an Unmethylated Fragment Ratio (UFR) value or both a MFR value and an UFR value, wherein the MCBs are based on CpGs pre-determined within the DNA; and
(h) calculating a p-value for each of selected differential MCBs,

wherein the selected differential MCBs are selected based on pre-determined MFR and UFR values, and
wherein the p-value is based on a pre-determined baseline distribution of MFR values if selected differential MCBs are hypermethylated or UFR values if selected differential MCBs are hypomethylated; and

(i) calculating a methylation score using the equation

$$Score_n = \frac{-2 \sum_{j=1}^{J} c_j \cdot \ln p_{n,j}}{\sum_{j=1}^{J} c_j}$$

wherein $c_j$ is the weight of $MCB_j$ within $sample_n$, $p_{n,j}$ is the p-value of (h) for $MCB_j$ in $sample_n$, wherein $sample_n$ has $J$ number of MCB, and wherein the methylation score is a hypermethylation score if selected differential MCBs in (h) are hypermethylated and is a hypomethylation score if selected differential MCBs in (h) are hypomethylated and is a hybrid methylation score if selected differential MCBs in (h) comprise both hypermethylated and hypomethylated MCBs.

2. The method of aspect 1, wherein selected differential MCBs in (h) are hypermethylated and wherein a hypermethylation score is calculated in (i).

3. The method of aspect 1, wherein selected differential MCBs in (h) are hypomethylated wherein a hypomethylation score is calculated in (i).

4. The method of aspect 1, wherein selected differential MCBs in (h) comprise both hypermethylated and hypomethylated MCBs and wherein a hybrid methylation score is calculated in (i).

5. The method of any one of aspects 1-4, wherein the $c_j$ is equal to

$count_{n,j}$ or
$-count_{n,j} \cdot \ln FDR_j$,

wherein $count_{n,j}$ is the number of fragments from $sample_n$ on $MCB_j$, and $FDR_j$ is false discovery/positive rate of $MCB_j$.

6. The method of any one of aspects 1-5, wherein the sample is a plasma sample.

7. The method of aspect 6, wherein the DNA is cell-free DNA.

8. A method of treating a subject having cancer, the method comprising:

(A) determining the methylation score of DNA of a test subject according to the method of any one of aspects 1-7; and
(B) determining that the test subject has cancer based on the methylation score of (A); and
(C) treating the test subject.

9. A method for determining a ctDNA Fraction (CTDF) value, the method comprising:

(a) providing a sample from a subject;
(b) isolating DNA from the sample of (a);
(c) treating isolated DNA of (b) with bisulfite or enzyme to perform conversion of unmethylated cytosines in the DNA;
(d) performing library construction of the converted DNA of (c) by paired end next generation sequencing (NGS);
(e) obtaining sequencing data from the paired end NGS of (d) and determining DNA sequences of DNA fragments present,
wherein the sequence of a DNA fragment is determined by merging the sequences of read-pairs for the DNA fragment;
(f) identifying methylation status of DNA fragments of (e) by comparing a reference genome to the sequencing data of (e) to determine if a cytosine base in a CpG site within a DNA fragment is methylated or unmethylated;
(g) calculating for Methylation-Correlated Blocks (MCBs) a Methylated Fragment Ratio (MFR) value, wherein the MCBs are based on CpGs pre-determined within the DNA; and
(h) calculating tumor and non-tumor likelihood values for each of selected differential MCBs,

wherein the selected differential MCBs are selected based on pre-determined MFR values, and
wherein the tumor and non-tumor likelihood values are based on a pre-determined beta distribution of MFR values calculated in (g); and

(i) calculating a ctDNA Fraction (CTDF) value based on the tumor and non-tumor likelihood values determined in (h) using the equation

$$\log P(F|\theta, M) = \sum_c w_c \cdot \log(\theta \cdot P(f^c|m^T_j) + (1 - \theta) \cdot P(f^c|m^N_j))$$

wherein

$j$ is the MCB covered by $f^c$;
$P(f^c|m^T_j)$ and $P(f^c|m^N_j)$ are

$$P(f|m^T_j) = \prod_h \frac{B(f_h + \alpha^T_j, 1 - f_h + \beta^T_j)}{B(\alpha^T_j, \beta^T_j)}$$

and

$$P(f|m^N_j) = \prod_h \frac{B(f_h + \alpha^N_j, 1 - f_h + \beta^N_j)}{B(\alpha^N_j, \beta^N_j)}$$

respectively, for a given fragment $f^c$;
$\alpha^T_j$, $\beta^T_j$, $\alpha^N_j$ and $\beta^N_j$ are parameters of tumor or normal class beta distributions of MFR on MCB $j$, which is estimated from $m^T_j$ and $m^N_j$;
$m^T_j$ is the tumor class methylation pattern on $MCBj$ and $m^N_j$ is the normal class methylation pattern on MCB$j$;
$f_h$ is 0 or 1;
$\theta$ is estimated by a grid search;
and $w_c$ is the weight assigned for $f^c$.

10. The method of aspect 9, wherein $w_c$ is one of

| |
|---|
| $MR$ |
| $MR^2$ |
| $\sqrt{MR}$ |
| $\dfrac{1}{\log MR}$ |
| $\begin{cases} 1, & MR \geq MR_b \| MR \leq MR_a \\ 0, & MR_a < MR < MR_b \end{cases}$ |

wherein $MR$ is the percentage of methylated CpGs of each fragment, $MR_b$ is the threshold of $MR$ for methylated fragments, and $MR_a$ is the threshold of $MR$ for unmethylated fragments.

11. The method of aspect 9 or 10, wherein the sample is a plasma sample.

12. The method of aspect 11, wherein the DNA is cell-free DNA.

13. A method of treating a subject having cancer, the method comprising:

(A) determining the ctDNA Fraction (CTDF) value of a test subject according to the method of any one of aspects 9-12; and
(B) determining that the test subject has cancer based on the CTDF of (A); and
(C) treating the test subject.

It shall be noted that the preceding are merely examples of embodiments. Other exemplary embodiments are apparent from the entirety of the description herein. It will also be understood by one of ordinary skill in the art that each of these embodiments may be used in various combinations with the other embodiments provided herein.

The following examples further illustrate the invention but, of course, should not be construed as in any way limiting its scope.

EXAMPLE 1

This Example demonstrates calculation of Methylated Fragment Ratio (MFR) and Unmethylated Fragment Ratio (UFR), in accordance with embodiments of the invention.

1.1 Defining Methylation-Correlated Blocks (MCBs)

CpGs meeting the following three criteria are merged into an MCB:

(1) The distance between $CpG_i$ and $CpG_{i+1}$ is less than $Distance_{max}$, where $Distance_{max}$ is customized;
(2) The correlation between $CpG_i$ and $CpG_{i+1}$ is no less than $Correlation_{min}$, where the correlation between CpGs is measured by the Pearson Correlation Coefficient, and $Correlation_{min}$ is customized; and
(3) The minimum number of CpGs contained in an MCB is no less than $c_{min}$, where $c_{min}$ is customized.

To calculate the correlation between $CpG_i$ and $CpG_{i+1}$, beta-values of $CpG_i$ and $CpG_{i+1}$ of a group of samples, $\{sample_1, \cdots , sample_N\}$, are first calculated. Specifically, the Person Correlation Coefficient can be calculated by the following formula:

$$Cor_{i,i+1} = \frac{\sum_{n=1}^{N}(beta_{n,i} - \overline{beta}_i)(beta_{n,i+1} - \overline{beta}_{i+1})}{\sqrt{\sum_{n=1}^{N}(beta_{n,i} - \overline{beta}_i)^2}\sqrt{\sum_{n=1}^{N}(beta_{n,i+1} - \overline{beta}_{i+1})^2}}$$

where $Cor_{i,i+1}$ is the correlation between $CpG_i$ and $CpG_{i+1}$, $beta_{n,i}$ and $beta_{n,i+1}$ are the beta-values of sample $n$ on $CpG_i$ and $CpG_{i+1}$, $\overline{beta}_i$ and $\overline{beta}_{i+1}$ are the mean beta-values among $\{sample_1, \cdots , sample_N\}$ on $CpG_i$ and $CpG_{i+1}$.

With an increasing $c_{min}$, $Correlation_{min}$ or a decreasing $Distance_{max}$, MCBs with less strong correlations are filtered out, which means the signals on the remaining MCBs are more reliable. As number of MCBs becomes smaller, though, information can be lost. Parameters which balance the reliability and the amount of data can be selected.

1.2 Combining Read-Pairs into Fragments

Paired-end sequencing platforms read from both ends of ligated DNA fragments, and produce two reads for each sequence, R1 and R2.

These reads are de-duplicated and filtered according to their mapping qualities (using the program Bowtie2 (Langmead et al., Nature Methods, 9:357-359 (2012) and conversion rates. The conversion rate is computed using non-CpG Cs covered by the read:

$$Conversion\ rate = \frac{Number\ of\ non - CpG\ Cs\ read\ as\ T}{Number\ of\ non - CpG\ Cs}$$

If the conversion is successfully done, the conversion rate is 100%, and all of the non-CpG Cs should be read as Ts in the sequencing data.

After filtering reads with mapping qualities less than 20 and conversion rates less than 95%, the remaining read-pairs are merged into a fragment before analysis in order to restore the methylation status of the original DNA fragment comprehensively.

If R1 and R2 overlap and the certain bases are different from each other, bases on the read with a higher average quality score will be selected. If the overlapped bases are different and the average quality scores are equal, the selection will be random between R1 and R2.

1.3 Identifying Methylated Fragments of MCB

Methylation statuses of CpGs contained in an MCB are extracted and checked by fragment. For each MCB, fragments covering a minimum of $x_{min}$ CpGs on the MCB are included.

The joint-methylation-status of $H$ CpGs contained in $MCB_j$ of a fragment is denoted as $f = \{f_1, f_2, \cdots \}$, where the binary value $f_h$ is 0 or 1, representing methylated or unmethylated status of the CpG site $h$ in fragment $f$. Using the joint-methylation-status, the percentage of methylated CpGs of each fragment is computed as

$$MR = \frac{\sum_{h=1}^{H} f_h}{H}.$$

Fragments with $MR$ higher than or equal to $MR_b$ in $MCB_j$ are identified as methylated fragments, while those with $MR$ lower than or equal to $MR_a$ are identified as unmethylated fragments. The rest are categorized as intermediate fragments.

Parameter $x_{min}$ should be an integer no larger than $c_{min}$, described above in section 1.1. $MR_a$ and $MR_b$ should range from 0 to 1, while $MR_b$ should be larger than $MR_a$. Parameters can be adjusted according to user preference.

For the Examples, the values are $x_{min} = 3$, $MR_b = 1$, and $MR_a = 0$.

1.4 Calculating MFR and UFR of MCB

Under the criteria of section 1.3, $H$ fragments which cover a specific $MCB_j$ are divided into three groups: methylated, unmethylated and intermediate fragments.

Methylated Fragment Ratio (MFR) of $MCB_j$ is calculated by

$$MFR_j = \frac{count^M_{\ j}}{count^M_{\ j} + count^U_{\ j} + count^I_{\ j}}$$

and Unmethylated Fragment Ratio (UFR) of $MCB_j$ is

$$UFR_j = \frac{count^U_{\ j}}{count^M_{\ j} + count^U_{\ j} + count^I_{\ j}}$$

where $count^M_j = \sum_{h=1}^{H}(MR_h \geq MR_b)$ indicates the number of methylated fragments of $MCB_j$,

$count^U_j = \sum_{h=1}^{H}(MR_h \leq MR_a)$ indicates the number of unmethylated fragments of $MCB_j$,

$count^U_j = \sum_{h=1}^{H}(MR_a < MR_h < MR_b)$ indicates the number of unmethylated fragments of $MCB_j$.

EXAMPLE 2

This Example demonstrates the original Methylation Score Model, as described in Liu et al., Ann. Oncol., 29: 1445-1453 (2018).

2.1 Selecting Differential Hypermethylated CpGs

The first step of the Methylation Score Model is to find hypermethylated CpGs, which are defined as CpGs with higher methylation level in the case group than in the control group.
Commonly, moderated t-test is performed by using the "Limma" package from R to compare the methylation level between groups. Beta-values are logit-transformed to M-values before the test:

$$M_{n,i} = \log 2 \frac{beta_{n,i}}{1 - beta_{n,i}}$$

where $M_{n,i}$ is the M-value of $sample_n$ on $CpG_i$, and $beta_{n,i}$ is the beta-value of $sample_n$ on $CpG_i$. $p_i$ is the p-value of moderated t-test comparing the mean M-value of $CpG_i$ between cases and controls. $FDR_i$, the Benjamini-Hochberg critical value for $p_i$, is then computed to control the false discovery/positive rate (FDR).
To decide whether a CpG is hypermethylated or not, the difference of the mean beta-value between groups is calculated. The difference of $CpG_i$ is:

$$diff_i = \overline{beta^{case}}_i - \overline{beta^{control}}_i$$

where $\overline{beta^{case}}_i$ is the mean beta-value of $CpG_i$ among case group, while $\overline{beta^{control}}_i$ is the mean among control group.
If $FDR_i$ is smaller than 0.05 and $diff_i$ is positive and larger than the pre-defined cutoff $diff_{min}$, then $CpG_i$ is a differential hypermethylated CpG. It is selected as a marker for building the Methylation Score Model.

2.2 Generating Baseline Distributions of Beta-value

For each selected hypermethylated $CpG_i$, beta-values of control samples are assumed to follow a normal distribution with a mean of $\mu^{control}_i$ and a standard deviation of $\sigma^{control}_i$:

$$beta_{n,i} \sim Norm(\mu^{control}_i, \sigma^{control}_i)$$

where $\mu^{control}_i$ is the mean of beta-value of $CpG_i$ among control samples, and $\sigma^{control}_i$ is the standard deviation of beta-value of $CpG_i$ among control samples.

2.3 Computing Per-CpG P-value

With a known baseline distribution $Norm(\mu^{control}_i, \sigma^{control}_i)$ and a known beta-value $beta_{n,i}$, the Z-score of $sample_n$ on $CpG_i$ can be computed as $Z_{n,i} = \frac{beta_{n,i} - \mu^{control}_i}{\sigma^{control}_i}$. This Z-score is then transformed to a p-value $p_{n,i}$.

After repeating this process for $N$ samples and I CpGs, for each $sample_n$, a set of p-values $\{p_{n,1}, \cdots, p_{n,I}\}$ is obtained.

2.4 Computing Final Methylation Score

The final score of $sample_n$ is a weighted average of the log-transformed p-value fom section 2.3:

$$Score_n = \frac{-2\sum_{i=1}^{I} depth_{n,i} \cdot \ln p_{n,i}}{\sum_{i=1}^{I} depth_{n,i}}$$

where $depth_{n,i}$ is the sequencing depth of $sample_n$ on $CpG_i$.

This score indicates the overall difference of the methylation level between the tested sample and the baseline distribution. A higher score is associated with higher probability of being a cancer case. Cutoff can be set as the 95th percentile/maximum of the control group, or any rational value.

EXAMPLE 3

This Example demonstrates the Methylation Score Model modified in accordance with embodiments of the invention.

3.1 Selecting Differential MCBs

Markers in the modified model are not hypermethylated CpGs but hypermethylated MCBs. A similar selection procedure is performed on $J$ candidate MCBs defined in Example 1, section 1.1.

The methylation level of MCB can either be the mean beta-values of CpGs on MCB or MFR/UFR calculated as in Example 1, section 1.4.

If MFRs are used, moderated t-tests are performed on logit-transformed MFRs to generate FDRs, according to which differential MCBs can be selected. Differences between the mean case MFRs and mean control MFRs are used to determine the direction of differential MCBs.

Logit-transformed MFR of $sample_n$ on $MCB_j$:

$$logitMCB_{n,j} = \log 2 \frac{MFR_{n,j}}{1 - MFR_{n,j}}.$$

FDR of $MCB_j$ is $FDR_j$. The difference of $MCB_j$ is

$$diff_j = \overline{MFR^{case}}_j - \overline{MFR^{control}}_j.$$

If $FDR_j$ is smaller than 0.05 and $diff_j$ is positive and larger than the pre-defined cutoff $diff_{min}$, $MCB_j$ is a differential hypermethylated MCB and is selected as a marker for the modified model. $\overline{MFR^{control}}_j$ and $\overline{MFR^{case}}_j$ are mean MFR of $MCB_j$ in control and case groups, respectively.

Although hypermethylated MCBs are selected here, the model is applicable to a global hypomethylated pattern in tumor cells: If the data is generated using a hypomethylation panel, hyponethylated MCBs can also be selected as markers. UFR instead of MFR will be used as the measurement of methylation level.

3.2 Generating Baseline Distributions of MFR

The original methylation model assumes normal distributions for beta-values of CpGs, but the natural distributions of beta-value are far from a normal distribution. For the model, the logit transformations of the methylation level measurement can be used.

MFR is used to measure the methylation level of hypermethylated MCB. For each selected hypermethylated $MCB_j$, logit-transformed MFRs of control samples are taken to follow a normal distribution with a mean of $\mu^{control}_j$ and a standard deviation of $\sigma^{control}_j$:

$$logitMCB_{n,j} \sim Norm(\mu^{control}_j, \sigma^{control}_j)$$

where $\mu^{control}_j$ and $\sigma^{control}_j$ are the mean and the standard deviation of logit-transformed MFR of $MCB_j$ among control samples.

If hypomethylated MCBs are used, methylation level of these MCBs will be measured by UFR. The distribution of logit-transformed UFRs among control samples is:

$$logitMCB_{n,j} \sim Norm(\mu^{control}{}_j, \sigma^{control}{}_j)$$

where $\mu^{control}{}_j$ and $\sigma^{control}{}_j$ are the mean and the standard deviation of logit-transformed UFR of hypomethylated $MCB_j$ among control samples.

Although a normal distribution for logit-MFR or logit-UFR is used here, this is not the only option. Other distributions such as beta distribution and Poisson distribution are good substitutes.

3.3 Computing Per-MCB P-value

With a known baseline distribution $Norm(\mu^{control}{}_j, \sigma^{control}{}_j)$ and a known $MFR_{n,j}$, the Z-score of $sample_n$ on $MCB_j$ can be computed by

$$Z_{n,j} = \frac{logitMFR_{n,j} - \mu^{control}{}_j}{\sigma^{control}{}_j}.$$

This Z-score is then transformed to a p-value $p_{n,j}$.

After repeating this process for $N$ samples and $J$ MCBs, a set of p-value $\{p_{n,1}, \cdots, p_{n,J}\}$ are obtained for each $sample_n$.

If hypomethylated MCBs are used, computation of p-value is almost the same as above, except instead of $MFR_{n,j}$, $UFR_{n,j}$ is used to calculate the Z-score of $sample_n$ on $MCB_j$. Baseline distribution of UFR in hypomethylated $MCB_j$ is $Norm(\mu^{control}{}_j, \sigma^{control}{}_j)$, and Z-score is computed by

$$Z_{n,j} = \frac{logitUFR_{n,j} - \mu^{control}{}_j}{\sigma^{control}{}_j}.$$

The baseline distribution is not necessarily a normal distribution. If any distribution other than normal distribution is used, the p-value calculation will be changed correspondingly.

3.4 Computing Final Methylation Score

The final score of $sample_n$ is a weighted average of the log-transformed p-value set from section 3.3:

$$Score_n = \frac{-2 \sum_{j=1}^{J} c_j \cdot \ln p_{n,j}}{\sum_{j=1}^{J} c_j}$$

where $c_j$ is the weight of $MCB_j$, which can optionally be $count_{n,j}$, the number of fragments on $MCB_j$ from $sample_n$.

This score indicates the overall difference of the methylation level between the tested sample and the control group. A higher score is associated with higher probability of being a cancer case. Cutoff can be set as the 95th percentile, the maximum of the control group, or any rational value.

3.5 Alternative Weights in Score Calculation

In section 3.4, the final methylation score is a weighted average of the MCB-level p-value. The number of fragments are used as weight, based on the assumption that each fragment contributes equally to the score calculation. Therefore, weights of MCBs with higher coverage are higher.

From another perspective, weights of MCBs can be assigned according to their importance. Since methylation score is interpreted as the overall difference of the methylation level from the control group, importance of an MCB can be equated with the difference between cases and controls. In section 3.1, when selecting differential MCBs, the FDR and the mean methylation level difference between groups for each MCB were computed. Weight of an MCB can either depend on FRD or on mean difference between groups. For example, $abs(diff)_j$ or $-\ln FDR_j$ for $MCB_j$.

Taking into account both the fragment-level contribution and the MCB-level importance, a combined weight can be used, such as $-count_{n,j} \cdot \ln FDR_j$.

EXAMPLE 4

This Example demonstrates the original CancerDetector Model, as described in Li et al., Nuc. Acids Res., 46: e89 (2018).

4.1 Selecting Frequently Differential Methylation Regions (FDMR)

CpGs are grouped into CpG clusters before marker selection. Two adjacent CpG sites are grouped into a CpG cluster if their flanking regions (100 bp up- and downstream) overlap.

These CpG clusters are candidates for FDMR and are further refined:

(1) At least three CpG sites (in the microarray data) are included in a cluster to obtain a robust measurement of methylation values in the solid tumor samples;
(2) The cluster is reasonably sized; and
(3) As many clusters that span within a type of genomic region (either CpG islands or shores) as possible are kept.

Since CancerDetector is designed for low coverage sequencing data like Whole Genome Bisulfite Sequencing (WGBS) and Reduced Representation Bisulfite Sequencing (RRBS), in order to obtain reliable values, the methylation level is calculated by CpG clusters and is defined as the average methylation level of all CpG sites in the cluster. This means the methylation levels of CpGs in same CpG cluster are even.

FDMRs are selected from CpG clusters, and should meet the following criteria:

(1) Methylation statuses are differential between matched tumor and normal tissues in more than half of the matched pairs; and
(2) The difference between the medians of its methylation levels in two classes is greater than a cutoff.

4.2 Building Beta-value Distributions

Given a region, the methylation levels of all samples in a class are modeled to follow a beta distribution. Distribution of the methylation level on $FDMR_k$ is $Beta(\alpha^T{}_k, \beta^T{}_k)$ in the tumor class and $Beta(\alpha^N{}_k, \beta^N{}_k)$ in the normal class. The parameters of a Beta distribution can be determined from the sample population of a class, using either the method of moments or maximum likelihood.

4.3 Calculating Per-read Likelihood

Each cfDNA read is classified as normal class or tumor class based on the joint-methylation-status of multiple CpG sites contained in FDMR on that read. The joint-methylation-status in a cfDNA read is denoted as $r = \{r_1, r_2, \cdots\}$, where the binary value $r_h$ is 0 or 1, representing methylated or unmethylated status of the CpG site $h$ in read $r$. The binary vector $r$ is modeled by the Beta-Bernoulli distribution.

Given the tumor class methylation pattern $m^T{}_k$ of FDMR $k$, the tumor class likelihood of read r can be calculated as below:

$$P(r|m^T{}_k) = \prod_h P(r_v|Beta(\alpha^T{}_v, \beta^T{}_v)) = \prod_h \frac{B(r_v + \alpha^T{}_v, 1 - r_v + \beta^T{}_v)}{B(\alpha^T{}_v, \beta^T{}_v)}$$

where $B(x, y)$ is the beta function, $v$ represents CpG site $v$ in FDMR $k$, $\alpha^T{}_v$ and $\beta^T{}_v$ are parameters of the tumor class beta distribution of CpG $v$ estimated from $m^T{}_k$.

As defined in section 4.1, the methylation level of a FDMR is defined as the average methylation level of all CpG sites in the region. Therefore, the parameter of CpG $v$ can also be denoted as $\alpha^T{}_k$ and $\beta^T{}_k$. The formula is then translated into:

$$P(r|m^T{}_k) = \prod_h \frac{B(r_k + \alpha^T{}_k, 1 - r_j + \beta^T{}_k)}{B(\alpha^T{}_k, \beta^T{}_k)}$$

Similarly, with the normal class methylation pattern $m^N{}_k$, the normal class likelihood of read r is:

$$P(r|m^N{}_k) = \prod_h \frac{B(r_k + \alpha^N{}_k, 1 - r_j + \beta^N{}_k)}{B(\alpha^N{}_k, \beta^N{}_k)}$$

where $\alpha^N{}_k$ and $\beta^N{}_k$ are parameters of the normal class beta distribution of FDMR $k$ estimated from $m^N{}_k$.

### 4.4 Estimating ctDNA Fraction (CTDF)

Methylation pattern of all $K$ FDMR is denoted as $M = \{(m^T{}_k, m^N{}_k)\}$, $k = 1, \ldots, K$. The binary vector set of $C$ reads covering FDMRs is denoted as $R = \{r^c\}$. Reads are assumed to be from one of the two classes, the tumor class or the normal class. CTDF $\theta (0 < \theta < 1)$ is estimated by maximizing the log-likelihood, which is calculated by the formula:

$$\log P(R|\theta, M) = \sum_c \log(\theta \cdot P(r^c|m^T{}_k) + (1 - \theta) \cdot P(r^c|m^N{}_k))$$

where $k$ is the FDMR where $r^c$ covers, $P(r^c|m^T{}_k)$ and $P(r^c|m^N{}_k)$ are calculated as in section 4.3.

Estimation of $\theta$ is done by a grid search. One thousand one fraction values uniformly distributed between 0% and 100% are exhaustively enumerated to find the global optimization.

### EXAMPLE 5

This Example demonstrates the CancerDetector Model modified in accordance with embodiments of the invention.

### 5.1 Selecting Differential MCBs

Markers in the modified model are MCBs, not FDMR. The procedure is the same as in Example 3, section 3.1.

### 5.2 Building MFR Distributions

MFR distributions are built in the modified model. MFRs can be modeled by beta distributions.

Distribution of MFR on $MCB_j$ is $Beta(\alpha^T{}_j, \beta^T{}_j)$ in tumor class and $Beta(\alpha^N j, \beta^N{}_j)$ in normal class. The parameters are determined from the tumor class samples and from the normal class samples, respectively.

### 5.3 Calculating Per-fragment Likelihood

Similar as the Modified Methylation Score Model, paired reads are firstly merged into fragments, according to the protocol in Example 1, section 1.2. This means that the likelihood is now calculated by fragment, not by read.

The joint-methylation-status in a cfDNA fragment is denoted as $f = \{f_1, f_2, \cdots \}$, where the binary value $f_h$ is 0 or 1, representing methylated or unmethylated status of the CpG site $h$ in fragment $f$. The binary vector $f$ is modeled by the Beta-Bernoulli distribution.

Given the tumor and normal class methylation pattern $m^T{}_j$ and $m^N{}_j$ on MCB $j$, the tumor and normal class likelihoods of fragment $f$ are:

$$P\big(f|m^T{}_j\big) = \prod_h \frac{B(f_h + \alpha^T{}_j, 1 - f_h + \beta^T{}_j)}{B(\alpha^T{}_j, \beta^T{}_j)}$$

and

$$P\big(f|m^N{}_j\big) = \prod_h \frac{B(f_h + \alpha^N{}_j, 1 - f_h + \beta^N{}_j)}{B(\alpha^N{}_j, \beta^N{}_j)}$$

where $\alpha^T{}_j, \beta^T{}_j, \alpha^N{}_j$ and $\beta^N{}_j$ are parameters of tumor or normal class beta distributions of MFR on MCB $j$, which is estimated from $m^T{}_j$ and $m^N{}_j$.

5.4 ctDNA Fraction (CTDF)

Methylation pattern of all $J$ MCBs is denoted as $M = \{(m^T_j, m^N_j)\}, j = 1, \ldots, J$. The binary vector set of $C$ fragments covering MCBs is denoted as $F = \{f^c\}$.

Similar to the original CancerDetector Model, CTDF $\theta (0 < \theta < 1)$ can be estimated by a maximum likelihood estimation (MLE) method by maximizing the global log-likelihood, but here weights are added as the coefficients of per-fragment log-likelihood. The weighted log-likelihood is calculated by the following formula:

$$\log P(F|\theta, M) = \sum_c w_c \cdot \log(\theta \cdot P(f^c|m^T_j) + (1 - \theta) \cdot P(f^c|m^N_j))$$

where $j$ is the MCB covered by $f^c$, $P(f^c|m^T_j)$ and $P(f^c|m^N_j)$ are calculated in section 5.3, $w_c$ is the weight assigned for $f^c$.

To reduce the noise caused by technical artifacts, weight of an intermediate fragment is set to a lower value than a fully methylated/unmethylated fragment. Here are some examples of weights that can be used:

|   | | Weight |
|---|---|---|
| A | | $MR$ |
| B | | $MR^2$ |
| C | | $\sqrt{MR}$ |
| D | | $\dfrac{1}{\log MR}$ |
| E | | $\begin{cases} 1, & MR \geq MR_b \mid MR \leq MR_a \\ 0, & MR_a < MR < MR_b \end{cases}$ |

where $MR$ is the percentage of methylated CpGs on MCB of a fragment ($MR$ is defined in Example 1, section 1.3).

Weight E is applied in the dataset, $MR_a$=0 and $MR_b$=1 as Example 1, section 1.3, because it can improve the model performance. The following values can be set: $w = 1$ for fully methylated/unmethylated fragments, and $w = 0$ for intermediate methylated fragments.

Estimation of $\theta$ is done by grid search. Ten fraction values uniformly distributed between 0% and 0.1% plus 1000 fraction values uniformly distributed between 0.1% and 100% are exhaustively enumerated to find the global optimization.

5.5 Alternative Weights in CTDF Estimation

In section 5.4, weights are assigned to fragments depending on their $MRs$. These weights are to reduce technical artifacts. Beside technical artifacts, the more CpGs a fragment covers, the more information it provides. As in Example 3, section 3.5, statistical values of MCBs, such as FDR and mean difference, reflect the methylation level difference between groups, implying the importance of MCBs.

Therefore, an example of the updated weight E in section 5.4 is:

$$\begin{cases} -H \cdot \ln FDR_j, & MR \geq MR_b \mid MR \leq MR_a \\ 0, & MR_a < MR < MR_b \end{cases}$$

where $H$ is the number of CpGs in $MCB_j$ covered by the fragment, $FDR_j$ is the FDR computed for $MCB_j$.

In this case, intermediate methylated fragments are not used, weights of methylated and unmethylated fragments are associated with the importance of the MCB (defined as the significance of difference between groups) and the number of covered CpGs in that MCB.

EXAMPLE 6

This Example demonstrates use of the inventive methods, in accordance with embodiments of the invention.

Four samples of plasma from lung cancer patients with high CTDF were diluted at a rate of 1:27, 1:81, and 1:243 respectively. The samples underwent target sequencing by enzyme-based conversion. Additionally, to build the models, DNA extracted from 50 healthy plasma samples and 195 Formalin-fixed Paraffin-embedded (FFPE) tissues were converted and sequenced using the same panel. Among the 195 FFPE samples, 11 were from lung tumors.

Correlations of methylation level between adjacent CpGs were measured using the beta-values of the 195 FFPE samples. MCBs were defined as in Example 1, section 1.1, setting $Distance_{max}$=100 bp, $Correlation_{min}$=0.95 and $c_{min}$=3. MFR of the MCBs of the diluted plasmas and healthy plasmas were calculated as in Example 1, section 1.2-1.4, setting $x_{min}$ = 3, $MR_b$ = 1, and $MR_a$ =0. Strategy for MCB marker selection was described in Example 3, section 3.1. The mean beta-values of each MCB between the 11 lung cancer tissues and the 50 healthy plasmas were compared and only hypermethylated MCBs with FDR less than 0.05 and $diff_{min}$ larger than 0.1 were kept. Furthermore, to ensure that the methylation level was low in the healthy plasma, mean beta-values less than 0.02 in healthy plasmas was required. 208 MCBs met the criterion were selected as markers.

Fifty healthy plasma samples were used to build the baseline in the Methylation Score Model and the normal class baseline in the CancerDetector Model. Eleven lung cancer tissues were used to build the tumor class baseline in the CancerDetector Model.

To verify the superiority of MFR, the performance between Model A (TABLE 1) and B (TABLE 2) was compared: Model A - modified Methylation Score Model using MFR as the measure of methylation level and the number of fragments as the weight, Model B - modified Methylation Score Model using the mean beta-value of CpGs on an MCB as the measure of methylation level and the mean depth as the weight.

The performance between Model C (TABLE 3) and Model D (TABLE 4) was compared: Model C - modified CancerDetector Model with weight $\begin{cases} 1, & MR \geq MR_b | MR \leq MR_a \\ 0, & MR_a < MR < MR_b \end{cases}$. The parameters of baseline distributions of an MCB were tuned using the mean beta-value of CpGs on the MCB. Model D - modified CancerDetector Model without assigning weights. The parameters of baseline distributions of an MCB were tuned using the mean beta-value of CpGs on the MCB. Using the highest predicted value in the healthy individuals as the cutoff, samples with a value exceeding the cutoff were predicted as cancer cases and are bold and italicized with an asterisk in the tables below.

TABLE 1: Methylation Score Model (Original)

|  | 1/27 | 1/81 | 1/243 |
|---|---|---|---|
| Person 1 | 1.96 | 1.45 | 1.23 |
| Person 2 | *6.06\** | 2.02 | 1.12 |
| Person 3 | 1.18 | 0.83 | 0.68 |
| Person 4 | 3.62 | 1.41 | 0.94 |

TABLE 2: Methylation Score Model (Modified - MFR)

|  | 1/27 | 1/81 | 1/243 |
|---|---|---|---|
| Person 1 | *5.28\** | 2.86 | 1.48 |
| Person 2 | *15.12\** | *10.64\** | *4.10\** |
| Person 3 | *5.02\** | 2.13 | 2.01 |
| Person 4 | *11.10\** | *5.44\** | 2.82 |

TABLE 3: CancerDetector Model (Original)

|  | 1/27 | 1/81 | 1/243 |
|---|---|---|---|
| Person 1 | 1.60% | 1.00% | 0.79% |
| Person 2 | 3.40% | 1.70% | 1.00% |
| Person 3 | 1.20% | 1.00% | 0.83% |
| Person 4 | 2.20% | 1.10% | 0.85% |

TABLE 4: CancerDetector Model (Modified)

|  | 1/27 | 1/81 | 1/243 |
|---|---|---|---|
| Person 1 | *0.41%** | 0.18% | 0.09% |
| Person 2 | *1.20%** | *0.47%** | 0.18% |
| Person 3 | 0.21% | 0.12% | 0.10% |
| Person 4 | *0.87%** | *0.28%** | 0.16% |

It was found that the highest Methylation Score in healthy individuals was reduced from 6.92 to 4.36, the highest CTDF of healthy individuals estimated by CancerDetector reduced from 6.5% to 0.32%, and the sensitivity of both models were greatly improved.

EXAMPLE 7

This Example demonstrates use of the inventive methods, in accordance with embodiments of the invention.

Four samples of plasma from cancer patients with high CTDF were diluted at a rate of 1:1, 1:3, 1:9, 1:27, 1:81, and 1:243 respectively. The samples underwent target sequencing by bisulfite conversion. Additionally, to build the models, DNA extracted from 41 healthy plasmas, 35 lung cancer plasmas and 59 FFPE lung tissues were converted and sequenced using the same panel.

Methylation level between adjacent CpGs were measured using the beta-values of the 59 FFPE samples. MCBs were defined as in Example 1, section 1.1, setting $Distance_{max}$=100 bp, $Correlation_{min}$=0.95 and $c_{min}$=3. MFR of the MCBs of the diluted plasmas and healthy plasmas were calculated as in Example 1, section 1.2-1.4, setting $x_{min}$ = 3, $MR_b$ = 1, and $MR_a$ =0.

Strategy for MCB marker selection is as described in Example 3, section 3.1. The mean beta-values of each MCB between the 35 lung cancer plasmas and the 41 healthy plasmas were compared and only hypermethylated MCBs with FDR less than 0.05 and $diff_{min}$ larger than 0.1 were kept. Furthermore, to ensure that the methylation level was low in the healthy plasma, the mean beta-values of MCB was required to be less than 0.02 in over 80% healthy plasmas. Twenty-one MCBs met the criterion were selected as markers.

Forty-one healthy plasmas were used to build the baseline in the Methylation Score Model and the normal class baseline in the CancerDetector Model. Thirty-fove lung cancer plasmas were used to build the tumor class baseline in the CancerDetector Model.

The performance between Model A (TABLE 5) and B (TABLE 6) was compared: Model A - modified Methylation Score Model using MFR as the measure of methylation level and the number of fragments as the weight, Model B - modified Methylation Score Model using the mean beta-value of CpGs on an MCB as the measure of methylation level and the mean depth as the weight.

The performance between Model C (TABLE 7) and Model D (TABLE 8) was compared: Model C - modified CancerDetector Model with weight $\begin{cases} 1, & MR \geq MR_b | MR \leq MR_a \\ 0, & MR_a < MR < MR_b \end{cases}$. The parameters of baseline distributions of an MCB were tuned using the mean beta-value of CpGs on the MCB, Model D - modified CancerDetector Model without assigning weights. The parameters of baseline distributions of an MCB were tuned using the mean beta-value of CpGs on the MCB.

Using the highest predict value in the healthy individuals as the cutoff, samples with a value exceeding the cutoff were predicted as cases and are bold and italicized with an asterisk in the tables below .

TABLE 5: Methylation Score Model (Original)

|  | 1/1 | 1/3 | 1/9 | 1/27 | 1/81 | 1/243 |
|---|---|---|---|---|---|---|
| Person 1 | *57.10** | *19.44** | *10.99** | *6.54** | 4.65 | 3.80 |
| Person 2 | *50.35** | *36.96** | *21.46** | *11.75** | *16.25** | 3.84 |
| Person 3 | *49.90** | *10.02** | 5.63 | 4.31 | 3.54 | 3.57 |
| Person 4 | *62.01** | *28.69** | *20.65** | 5.90 | 5.22 | 4.16 |

TABLE 6: Methylation Score Model (Modified - MFR)

| | 1/1 | 1/3 | 1/9 | 1/27 | 1/81 | 1/243 |
|---|---|---|---|---|---|---|
| Person 1 | *36.69** | *30.05** | *18.19** | *13.02** | *13.91** | 2.09 |
| Person 2 | *55.62** | *48.81** | *32.85** | *17.52** | *12.73** | 4.62 |
| Person 3 | *28.26** | *19.32** | 6.35 | 2.56 | 1.20 | 2.86 |
| Person 4 | *48.46** | *43.10** | *32.64** | *11.71** | *6.53** | 0.94 |

TABLE 7: CancerDetector Model (Original)

| | 1/1 | 1/3 | 1/9 | 1/27 | 1/81 | 1/243 |
|---|---|---|---|---|---|---|
| Person 1 | *40.80%** | *11.90%** | *6.90%** | 3.80% | 1.80% | 1.30% |
| Person 2 | *78.90%** | *33.90%** | *11.60%** | 4.90% | 4.30% | 1.20% |
| Person 3 | *19.00%** | *6.80%** | 2.80% | 1.00% | 1.30% | 1.50% |
| Person 4 | *73.30%** | *19.50%** | *7.30%** | 3.20% | 1.90% | 0.50% |

TABLE 8: CancerDetector Model (Modified)

| | 1/1 | 1/3 | 1/9 | 1/27 | 1/81 | 1/243 |
|---|---|---|---|---|---|---|
| Person 1 | *31.30%** | *7.50%** | *3.30%** | *1.10%** | 0.55% | 0.08% |
| Person 2 | *70.60%** | *28.00%** | *8.60%** | *2.10%** | *1.40%** | 0.32% |
| Person 3 | *10.70%** | *3.00%** | 0.76% | 0.33% | 0.11% | 0.14% |
| Person 4 | *64.50%** | *13.40%** | *4.60%** | *1.30%** | 0.55% | 0.01% |

It was found that the highest Methylation Score in healthy individuals changed from 6.3 to 6.46, which was possibly due to the small-size marker-set; the highest CTDF of healthy individuals estimated by CancerDetector reduced from 4.9% to 0.8%; and sensitivity of both models were improved, although the cutoff of the Methylation Score Model increased.

EXAMPLE 8

This Example demonstrates use of the inventive methods, in accordance with embodiments of the invention.
Samples compared were RRBS plasma samples from a published paper (Guo et al. Nature Genetics, 49:635-642 (2017). Different from target sequencing, Reduced Representation Bisulfite Sequencing (RRBS) generates sequencing data which covers a broader region with lower depth (10-20x in this dataset).
RRBS data of 30 healthy, 20 cancer plasmas (10 of lung and colon cancer respectively) and 10 FFPE tissues (5 of lung and colon respectively) were downloaded to build and test the model.
Correlations of methylation level between adjacent CpGs were measured using the beta-values of 10 FFPE samples. MCBs were defined as in Example 1, section 1.1, setting $Distance_{max}$=100 bp, $Correlation_{min}$=0.7 and $c_{min}$=3. MFR of the MCBs of the diluted plasmas and healthy plasmas were calculated as in Example 1, section 1.2-1.4, setting $x_{min}$ = 3, $MR_b$ = 1, and $MR_a$ =0.
Strategy for MCB marker selection is as described in Example 3, section 3.1. For each cancer type, the mean beta-values between the 5 tumor tissues and 15 of the 30 healthy plasmas were compared and those kept were only MCBs with FDR less than 0.05, $diff_{min}$ larger than 0.4 and with average MCB mean beta-value less than 0.05 in healthy plasmas. Fiftyfour and 70 MCBs met the criteria and were selected as colon cancer and lung cancer markers respectively.
The performance between Model A and B (Figure 1) was compared: Model A - modified Methylation Score Model using MFR as the measure of methylation level and the number of fragments as the weight, Model B - modified Methylation Score Model using the mean beta-value of CpGs on an MCB as the measure of methylation level and the mean depth as the weight.
The performance between Model C and Model D (Figure 2) was compared: Model C - modified CancerDetector

Model with weight $\begin{cases} 1, & MR \geq MR_b | MR \leq MR_a \\ 0, & MR_a < MR < MR_b \end{cases}$ . The parameters of baseline distributions of an MCB were tuned using the mean beta-value of CpGs on the MCB, Model D - modified CancerDetector Model without assigning weights. The parameters of baseline distributions of an MCB were tuned using the mean beta-value of CpGs on the MCB.

It was found that AUC of Methylation Model was improved from less than 70% to 90%, and AUC of CancerDetector Model was improved as well.

Even though RRBS data differs greatly from target sequencing data, the inventive methods worked effectively.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

**Claims**

1. A method for determining a methylation score of DNA, the method comprising:

    (b) isolating DNA from a sample obtained from a subject;
    (c) treating isolated DNA of (b) with bisulfite or enzyme to perform conversion of unmethylated cytosines in the DNA;
    (d) performing library construction of the converted DNA of (c) by paired end next generation sequencing (NGS);
    (e) obtaining sequencing data from the paired end NGS of (d) and determining DNA sequences of DNA fragments present,
    wherein the sequence of a DNA fragment is determined by merging the sequences of read-pairs for the DNA fragment;
    (f) identifying methylation status of DNA fragments of (e) by comparing a reference genome to the sequencing data of (e) to determine if a cytosine base in a CpG site within a DNA fragment is methylated or unmethylated;
    (g) calculating for Methylation-Correlated Blocks (MCBs) a Methylated Fragment Ratio (MFR) value or an Unmethylated Fragment Ratio (UFR) value or both a MFR value and an UFR value, wherein the MCBs are based on CpGs pre-determined within the DNA; and
    (h) calculating a p-value for each of selected differential MCBs,

        wherein the selected differential MCBs are selected based on pre-determined MFR and UFR values, and wherein the p-value is based on a pre-determined baseline distribution of MFR values if selected differential MCBs are hypermethylated or UFR values if selected differential MCBs are hypomethylated; and

    (i) calculating a methylation score using the equation

$$Score_n = \frac{-2\sum_{j=1}^{J} c_j \cdot \ln p_{n,j}}{\sum_{j=1}^{J} c_j}$$

    wherein $c_j$ is the weight of $MCB_j$ within $sample_n$, $p_{n,j}$ is the p-value of (h) for $MCB_j$ in $sample_n$,
    wherein $sample_n$, has $J$ number of MCB, and
    wherein the methylation score is a hypermethylation score if selected differential MCBs in (h) are hypermethylated and is a hypomethylation score if selected differential MCBs in (h) are hypomethylated and is a hybrid methylation score if selected differential MCBs in (h) comprise both hypermethylated and hypomethylated MCBs.

2. The method of claim 1, wherein selected differential MCBs in (h) are hypermethylated and wherein a hypermethylation score is calculated in (i).

3. The method of claim 1, wherein selected differential MCBs in (h) are hypomethylated wherein a hypomethylation score is calculated in (i).

4. The method of claim 1, wherein selected differential MCBs in (h) comprise both hypermethylated and hypomethylated MCBs and wherein a hybrid methylation score is calculated in (i).

5. The method of any one of claims 1-4, wherein the $c_j$ is equal to

   $count_{n,j}$, or
   $-count_{n,j} \cdot \ln FDR_j$,

   wherein $count_{n,j}$ is the number of fragments from $sample_n$ on $MCB_j$, and $FDR_j$ is false discovery/positive rate of $MCB_j$.

6. The method of any one of claims 1-5, wherein the sample is a plasma sample.

7. The method of claim 6, wherein the DNA is cell-free DNA.

8. A method for determining a ctDNA Fraction (CTDF) value, the method comprising:

   (b) isolating DNA from a sample obtained from a subject;
   (c) treating isolated DNA of (b) with bisulfite or enzyme to perform conversion of unmethylated cytosines in the DNA;
   (d) performing library construction of the converted DNA of (c) by paired end next generation sequencing (NGS);
   (e) obtaining sequencing data from the paired end NGS of (d) and determining DNA sequences of DNA fragments present,
   wherein the sequence of a DNA fragment is determined by merging the sequences of read-pairs for the DNA fragment;
   (f) identifying methylation status of DNA fragments of (e) by comparing a reference genome to the sequencing data of (e) to determine if a cytosine base in a CpG site within a DNA fragment is methylated or unmethylated;
   (g) calculating for Methylation-Correlated Blocks (MCBs) a Methylated Fragment Ratio (MFR) value, wherein the MCBs are based on CpGs pre-determined within the DNA; and
   (h) calculating tumor and non-tumor likelihood values for each of selected differential MCBs,

       wherein the selected differential MCBs are selected based on pre-determined MFR values, and
       wherein the tumor and non-tumor likelihood values are based on a pre-determined beta distribution of MFR values calculated in (g); and

   (i) calculating a ctDNA Fraction (CTDF) value based on the tumor and non-tumor likelihood values determined in (h) using the equation

   $$\log P(F|\theta, M) = \sum_c w_c \cdot \log(\theta \cdot P(f^c|m^T{}_j) + (1 - \theta) \cdot P(f^c|m^N{}_j))$$

   wherein

       $j$ is the MCB covered by $f^c$;
       $P(f^c|m^T{}_j)$ and $P(f^c|m^N{}_j)$ are

   $$P(f|m^T{}_j) = \prod_h \frac{B(f_h + \alpha^T{}_j, 1 - f_h + \beta^T{}_j)}{B(\alpha^T{}_j, \beta^T{}_j)}$$

       and

$$P(f|m^N{}_j) = \prod_h \frac{B(f_h + \alpha^N{}_j, 1 - f_h + \beta^N{}_j)}{B(\alpha^N{}_j, \beta^N{}_j)}$$

respectively, for a given fragment $f^c$;

$\alpha^T{}_j$, $\beta^T{}_j$, $\alpha^N{}_j$ and $\beta^N{}_j$ are parameters of tumor or normal class beta distributions of MFR on MCB $j$, which is estimated from $m^T{}_j$ and $m^N{}_j$;

$m^T{}_j$ is the tumor class methylation pattern on $MCBj$ and $m^N{}_j$ is the normal class methylation pattern on MCB$j$;

$f_h$ is 0 or 1;

$\theta$ is estimated by a grid search;

and $w_c$ is the weight assigned for $f^c$.

9. The method of claim 8, wherein $w_c$ is one of

| $MR$ |
| :---: |
| $MR^2$ |
| $\sqrt{MR}$ |
| $\dfrac{1}{\log MR}$ |
| $\begin{cases} 1, & MR \geq MR_b \mid MR \leq MR_a \\ 0, & MR_a < MR < MR_b \end{cases}$ |

wherein $MR$ is the percentage of methylated CpGs of each fragment, $MR_b$ is the threshold of $MR$ for methylated fragments, and $MR_a$ is the threshold of $MR$ for unmethylated fragments.

10. The method of claim 8 or 9, wherein the sample is a plasma sample.

11. The method of claim 10, wherein the DNA is cell-free DNA.

**Patentansprüche**

1. Verfahren zur Bestimmung eines Methylierungs-Scores von DNA, wobei das Verfahren umfasst:

(b) Isolieren von DNA aus einer Probe, die von einem Subjekt erhalten wurde;

(c) Behandeln der isolierten DNA aus (b) mit Bisulfit oder Enzym, um die Umwandlung von unmethylierten Cytosinen in der DNA durchzuführen;

(d) Durchführen einer Bibliothekskonstruktion der konvertierten DNA aus (c) mittels Paired-End-Next-Generation-Sequenzierung (NGS);

(e) Erhalten von Sequenzierungsdaten aus der Paired-End-NGS aus (d) und Bestimmen von DNA-Sequenzen vorhandener DNA-Fragmente, wobei die Sequenz eines DNA-Fragments durch Zusammenführen der Sequenzen von Read-Paaren für das DNA-Fragment bestimmt wird;

(f) Identifizieren von Methylierungsstatus der DNA-Fragmente aus (e) durch Vergleichen eines Referenzgenoms mit den Sequenzierungsdaten aus (e), um zu bestimmen, ob eine Cytosinbase in einer CpG-Site innerhalb des DNA-Fragments methyliert oder unmethyliert ist;

(g) Berechnen eines Methylierungsfragment-Verhältnis (MFR)-Werts oder eines Nicht-Methylierungsfragment-Verhältnis (UFR)-Werts oder sowohl eines MFR-Werts als auch eines UFR-Werts für Methylation-Correlated-Blocks (MCBs), wobei die MCBs auf innerhalb der DNA vorbestimmten CpGs basieren; und

(h) Berechnen eines p-Werts für jede der ausgewählten differentiellen MCBs,

wobei die ausgewählten differentiellen MCBs auf der Grundlage vorbestimmter MFR- und UFR-Werte ausgewählt werden, und

wobei der p-Wert auf einer vorbestimmten Basis-Verteilung von MFR-Werten bei hypermethylierten ausgewählten differentiellen MCBs oder von UFR-Werten bei hypomethylierten ausgewählten differentiellen MCBs basiert; und

(i) Berechnen eines Methylierungs-Scores unter Verwendung der folgenden Gleichung

$$Score_n = \frac{-2\sum_{j=1}^{J} c_j \cdot \ln p_{n,j}}{\sum_{j=1}^{J} c_j}$$

worin $c_j$ die Gewichtung von $MCB_j$ innerhalb $Probe_n$ und $p_{n,j}$ der p-Wert von (h) für $MCB_j$ in $Probe_n$, worin $Probe_n$ die Anzahl $J$ von MCB aufweist, und

wobei der Methylierungs-Score ein Hypermethylierungs-Score bei den hypermethylierten ausgewählten differentiellen MCBs in (h) darstellt, ein Hypomethylierungs-Score bei den hypomethylierten ausgewählten differentiellen MCBs in (h) darstellt und ein hybrider Methylierungs-Score bei den ausgewählten differentiellen MCBs in (h), die hypermethylierte und hypomethylierte MCBs umfassen, darstellt.

2. Verfahren nach Anspruch 1, wobei die ausgewählten differentiellen MCBs in (h) hypermethyliert sind, und wobei der Hypermethylierungs-Score in (i) berechnet wird.

3. Verfahren nach Anspruch 1, wobei die ausgewählten differentiellen MCBs in (h) hypomethyliert sind, und wobei der Hypomethylierungs-Score in (i) berechnet wird.

4. Verfahren nach Anspruch 1, wobei die ausgewählten differentiellen MCBs in (h) die hypermethylierten und hypomethylierten MCBs umfassen, und wobei der hybride Methylierungs-Score in (i) berechnet wird.

5. Verfahren nach einem der Ansprüche 1 bis 4, wobei das $c_j$ gleich

$count_{n,j}$ oder
$-count_{n,j}\} \cdot \ln FDR_j$ ist,

worin $count_{n,j}$ die Anzahl von Fragmenten aus $Probe_n$ an $MCB_j$ darstellt und $FDR_j$ eine False-Discovery / Positive-Rate von $MCB_j$ darstellt.

6. Verfahren nach einem der Ansprüche 1 bis 5, wobei die Probe eine Plasmaprobe ist.

7. Verfahren nach Anspruch 6, wobei die DNA zellfreie DNA ist.

8. Verfahren zur Bestimmung eines ctDNA-Fraktion (CTDF)-Werts, wobei das Verfahren umfasst:

(b) Isolieren von DNA aus einer Probe, die von einem Subjekt erhalten wurde;
(c) Behandeln der isolierten DNA aus (b) mit Bisulfit oder Enzym, um die Umwandlung von unmethylierten Cytosinen in der DNA durchzuführen;
(d) Durchführen einer Bibliothekskonstruktion der konvertierten DNA aus (c) mittels Paired-End-Next-Generation-Sequenzierung (NGS);
(e) Erhalten von Sequenzierungsdaten aus der Paired-End-NGS aus (d) und Bestimmen von DNA-Sequenzen vorhandener DNA-Fragmente,
wobei die Sequenz eines DNA-Fragments durch Zusammenführen der Sequenzen von Read-Paaren für das DNA-Fragment bestimmt wird;
(f) Identifizieren von Methylierungsstatus der DNA-Fragmente aus (e) durch Vergleichen eines Referenzgenoms mit den Sequenzierungsdaten aus (e), um zu bestimmen, ob eine Cytosinbase in einer CpG-Site innerhalb des DNA-Fragments methyliert oder unmethyliert ist;
(g) Berechnen eines Methylierungsfragment-Verhältnis(MFR)-Werts für Methylation-Correlated-Blocks (MCBs), wobei die MCBs auf innerhalb der DNA vorbestimmten CpGs basieren; und
(h) Berechnen von Tumor- und Nicht-Tumor-Wahrscheinlichkeitswerten für jede der ausgewählten differentiellen MCBs,

wobei die ausgewählten differentiellen MCBs auf der Grundlage vorbestimmter MFR-Werte ausgewählt werden, und

wobei die Tumor- und Nicht-Tumor-Wahrscheinlichkeitswerte auf einer vorbestimmten Betaverteilung der in (g) berechneten MFR-Werte basieren; und

(i) Berechnen eines ctDNA-Fraktion (CTDF)-Werts auf der Grundlage der in (h) bestimmten Tumor- und Nicht-Tumor-Wahrscheinlichkeitswerte unter Verwendung der folgenden Gleichung

$$\log P(F|\theta, M) = \sum_c w_c \cdot \log(\theta \cdot P(f^c|m^T{}_j) + (1 - \theta) \cdot P(f^c|m^N{}_j))$$

worin

$j$ der von $f^c$ abgedeckte MCB darstellt; wobei
$P(f^c|m^T{}_j)$ und $P(f^c|m^N{}_j)$ jeweils

$$P(f|m^T{}_j) = \prod_h \frac{B(f_h + \alpha^T{}_j, 1 - f_h + \beta^T{}_j)}{B(\alpha^T{}_j, \beta^T{}_j)}$$

und

$$P(f|m^N{}_j) = \prod_h \frac{B(f_h + \alpha^N{}_j, 1 - f_h + \beta^N{}_j)}{B(\alpha^N{}_j, \beta^N{}_j)}$$

, für ein gegebenes Fragment $f^c$, sind; wobei
$\alpha^T{}_j$, $\beta^T{}_j$, $\alpha^N{}_j$ und $\beta^N{}_j$ Parameter von Tumor- oder Normal-Klasse-Betaverteilungen von MFR an MCB $j$ sind, was aus $m^T{}_j$ und $m^N{}_j$ abgeschätzt wird; wobei
$m^T{}_j$ ein Tumor-Klasse-Methylierungsmuster an MCB$j$ ist und $m^N{}_j$ ein Normal-Klasse-Methylierungsmuster an MCB$j$ ist; wobei
$f_h$ 0 oder 1 ist; wobei
$\theta$ durch eine Rastersuche abgeschätzt wird;
und wobei $w_c$ eine für $f^c$ zugewiesene Gewichtung ist.

9. Verfahren nach Anspruch 8, wobei $w_c$ aus einem der Folgenden ausgewählt ist:

| $MR$ |
|:---:|
| $MR^2$ |
| $\sqrt{MR}$ |
| $\dfrac{1}{\log MR}$ |
| $\begin{cases} 1, & MR \geq MR_b \mid MR \leq MR_a \\ 0, & MR_a < MR < MR_b \end{cases}$ |

wobei $MR$ ein Prozentsatz der methylierten CpGs jedes Fragments ist, $MR_b$ ein Schwellenwert von $MR$ für methylierte Fragmente ist und $MR_a$ ein Schwellenwert von $MR$ für unmethylierte Fragmente ist.

10. Verfahren nach Anspruch 8 oder 9, wobei die Probe eine Plasmaprobe ist.

11. Verfahren nach Anspruch 10, wobei die DNA zellfreie DNA ist.

**Revendications**

1. Procédé de détermination d'un score de méthylation d'ADN, le procédé comprenant :

    (b) isoler l'ADN d'un échantillon prélevé sur un sujet ;
    (c) traiter l'ADN isolé de (b) avec du bisulfite ou une enzyme pour effectuer une conversion des cytosines non méthylées dans l'ADN ;
    (d) construire une banque de l'ADN converti de (c) par Séquençage de Génération Suivante (NGS) d'extrémité appariée ;
    (e) obtenir des données de séquençage à partir du NGS d'extrémité appariée de (d) et déterminer des séquences ADN des fragments ADN présents,
    dans lequel la séquence d'un fragment ADN est déterminée en fusionnant les séquences de paires de lecture du fragment ADN ;
    (f) identifier un état de méthylation des fragments ADN de (e) en comparant un génome de référence aux données de séquençage de (e) afin de déterminer si une base de cytosine dans un site CpG à l'intérieur d'un fragment ADN est méthylée ou non méthylée ;
    (g) calculer pour des Blocs Corrélés à Méthylation (MCB) une valeur de Rapport de Fragments Méthylés (MFR) ou une valeur de Rapport de Fragments Non méthylés (UFR) ou à la fois une valeur MFR et une valeur UFR, les MCB étant basés sur des CpG prédéterminés à l'intérieur de l'ADN ; et
    (h) calculer une valeur-p pour chacun des MCB différentiels sélectionnés,

        dans lequel les MCB différentiels sélectionnés sont sélectionnés en fonction des valeurs MFR et UFR prédéterminées, et
        dans lequel la valeur-p est basée sur une distribution de référence prédéterminée des valeurs MFR si les MCB différentiels sélectionnés sont hyperméthylés ou des valeurs UFR si les MCB différentiels sélectionnés sont hypométhylés ; et

    (i) calculer un score de méthylation à l'aide de l'équation

$$Score_n = \frac{-2\sum_{j=1}^{J} c_j \cdot \ln p_{n,j}}{\sum_{j=1}^{J} c_j}$$

    où $c_j$ est le poids de $MCB_j$ dans le $sample_n$, $p_{n,j}$ est la valeur-p de (h) pour $MCB_j$ dans le $sample_n$
    dans lequel le $sample_n$ a un nombre $J$ de MCB, et
    dans lequel le score de méthylation est un score d'hyperméthylation si les MCB différentiels sélectionnés de (h) sont hyperméthylés et est un score d'hypométhylation si les MCB différentiels sélectionnés de (h) sont hypométhylés et est un score de méthylation hybride si les MCB différentiels sélectionnés de (h) comprennent à la fois les MCB hyperméthylés et hypométhylés.

2. Procédé selon la revendication 1, dans lequel les MCB différentiels sélectionnés à (h) sont hyperméthylés et dans lequel un score d'hyperméthylation est calculé à (i).

3. Procédé selon la revendication 1, dans lequel les MCB différentiels sélectionnés à (h) sont hypométhylés, dans lequel un score d'hypométhylation est calculé à (i).

4. Procédé selon la revendication 1, dans lequel les MCB différentiels sélectionnés à (h) comprennent à la fois les MCB hyperméthylés et hypométhylés et dans lequel un score de méthylation hybride est calculé à (i).

5. Procédé selon l'une quelconque des revendications 1 à 4, dans lequel le $c_j$ est égal à

    $count_{n,j}$, ou
    $-count_{n,j} \cdot \ln FDR_j$,

dans lequel $count_{n,j}$ est le nombre de fragments à partir du $sample_n$ sur $MCB_j$, et $FDR_j$ est un taux de fausses découvertes/positifs de $MCB_j$.

**6.** Procédé selon l'une quelconque des revendications 1 à 5, dans lequel l'échantillon est un échantillon de plasma.

**7.** Procédé selon la revendication 6, dans lequel l'ADN est l'ADN acellulaire.

**8.** Procédé de détermination d'une valeur de Fraction d'ADNtc (CTDF), le procédé comprenant :

(b) isoler l'ADN d'un échantillon prélevé sur un sujet ;

(c) traiter l'ADN isolé de (b) avec du bisulfite ou une enzyme pour effectuer une conversion des cytosines non méthylées dans l'ADN ;

(d) construire une banque de l'ADN converti de (c) par Séquençage de Génération Suivante (NGS) d'extrémité appariée ;

(e) obtenir des données de séquençage à partir du NGS d'extrémité appariée de (d) et déterminer des séquences ADN des fragments ADN présents,

dans lequel la séquence d'un fragment ADN est déterminée en fusionnant les séquences de paires de lecture du fragment ADN ;

(f) identifier un état de méthylation des fragments ADN de (e) en comparant un génome de référence aux données de séquençage de (e) afin de déterminer si une base de cytosine dans un site CpG à l'intérieur d'un fragment ADN est méthylée ou non méthylée ;

(g) calculer pour des Blocs Corrélés à Méthylation (MCB) une valeur de Rapport de Fragments Méthylés (MFR), les MCB étant basés sur des CpG prédéterminés à l'intérieur de l'ADN ; et

(h) calculer des valeurs de vraisemblance tumorale et non tumorale pour chacun des MCB différentiels sélectionnés,

dans lequel les MCB différentiels sélectionnés sont sélectionnés en fonction des valeurs MFR prédéterminées, et

dans lequel les valeurs de vraisemblance tumorale et non tumorale sont basées sur une distribution bêta prédéterminée des valeurs MFR calculées à (g) ; et

(i) calculer une valeur de Fraction d'ADNtc (CTDF) en fonction des valeurs de vraisemblance tumorale et non tumorale déterminées à (h) à l'aide de l'équation

$$\log P(F|\theta, M) = \sum_c w_c \cdot \log(\theta \cdot P(f^c|m^T{}_j) + (1 - \theta) \cdot P(f^c|m^N{}_j))$$

dans lequel

$j$ est le MCB couvert par $f^c$ ;

$P(f^c|m^T{}_j)$ et $P(f^c|m^N{}_j)$ sont

$$P(f|m^T{}_j) = \prod_h \frac{B(f_h + \alpha^T{}_j, 1 - f_h + \beta^T{}_j)}{B(\alpha^T{}_j, \beta^T{}_j)}$$

et

$$P(f|m^N{}_j) = \prod_h \frac{B(f_h + \alpha^N{}_j, 1 - f_h + \beta^N{}_j)}{B(\alpha^N{}_j, \beta^N{}_j)}$$

respectivement, pour un fragment donné $f^c$ ;

$\alpha^T{}_j$, $\beta^T{}_j$, $\alpha^N{}_j$ et $\beta^N{}_j$ sont des paramètres de distributions bêta de classe tumorale ou normale de MFR sur MCB $j$, qui est estimée à partir de $m^T{}_j$ et $m^N{}_j$ ;

$m^T{}_j$ est le modèle de méthylation de classe tumorale sur le MCB $j$ et $m^N{}_j$ est le modèle de méthylation de classe normale sur le $MCB\ j$ ;

$f_h$ est égal à 0 ou 1 ;

$\theta$ est estimé par une recherche de grille ;

et $w_c$ est le poids attribué à $f^c$.

9.  Procédé selon la revendication 8, dans lequel $w_c$ est l'un de

| |
|:---:|
| $MR$ |
| $MR^2$ |
| $\sqrt{MR}$ |
| $\dfrac{1}{\log MR}$ |
| $\begin{cases} 1, & MR \geq MR_b \| MR \leq MR_a \\ 0, & MR_a < MR < MR_b \end{cases}$ |

dans lequel $MR$ est le pourcentage des CpG méthylés de chaque fragment, $MR_b$ est le seuil de $MR$ pour les fragments méthylés, et $MR_a$ est le seuil de $MR$ pour les fragments non méthylés.

10. Procédé selon la revendication 8 ou 9, dans lequel l'échantillon est un échantillon de plasma.

11. Procédé selon la revendication 10, dans lequel l'ADN est l'ADN acellulaire.

## Figure 1

Methylation Score Model

## Figure 2

### CancerDetector Model

Specificity (%)
Colorectal
Cancer

Specificity (%)
Lung Cancer

## REFERENCES CITED IN THE DESCRIPTION

**Patent documents cited in the description**

- WO 2018161031 A1

**Non-patent literature cited in the description**

- **KRISTINA WARTON et al.** Methylated circulating tumor DNA in blood: power in cancer prognosis and response. *ENDOCRINE RELATED CANCER*, 13 January 2016, vol. 23 (3), R157-R171
- **LIU L. et al.** *Targeted methylation sequencing of plasma cell-free DNA for cancer detection and classification*, 01 June 2018, vol. 29 (6), 1445-1453
- **LANGMEAD et al.** *Nature Methods*, 2012, vol. 9, 357-359
- **LIU et al.** *Ann. Oncol.*, 2018, vol. 29, 1445-1453
- **LI et al.** *Nuc. Acids Res.*, 2018, vol. 46, e89
- **GUO et al.** *Nature Genetics*, 2017, vol. 49, 635-642